# EUROPÄISCHE PATENTSCHRIFT

(11) **EP 0 975 638 B1**
(45) Veröffentlichungstag und Bekanntmachung des Hinweises auf die Patenterteilung: **07.08.2002**
(21) Anmeldenummer: 98912388.0
(22) Anmeldetag: 25.02.1998
(51) Int. Cl.: C07D 493/04

(54) **SEITENKETTENMODIFIZIERTE EPOTHILONE**
EPOTHILONES WITH A MODIFIED SIDE CHAIN
EPOTHILONES A CHAINE LATERALE MODIFIEE

(30) Priorität: 25.02.1997 DE 19707505
(43) Veröffentlichungstag der Anmeldung: 02.02.2000
(60) Teilanmeldung: 02001063.3
(73) Patentinhaber: Gesellschaft für Biotechnologische Forschung mbH (GBF), 38124 Braunschweig (DE)
(72) Erfinder: HÖFLE, Gerhard, D-38124 Braunschweig (DE); SEFKOW, Michael, D-14469 Potsdam (DE)
(74) Vertreter: Boeters, Hans Dietrich, Dr.
(86) Internationale Anmeldenummer: EP9801060
(87) Internationale Veröffentlichungsnummer: WO9838192

(56) Entgegenhaltungen:
- WO-A-93/10121
- K.C. NICOLAOU ET AL.: "Designed epothilones: combinatorial synthesis, tubulin assembly properties, and cytotoxic action against taxol-resistant tumr cells" ANGEWANDTE CHEMIE. INTERNATIONAL EDITION., Bd. 36, Nr. 19, 1997, WEINHEIM DE, Seiten 2097-2103, XP002070869

## Beschreibung

Epothilone A und B sind bekannt; vgl. beispielsweise DE 4 138 042, WO 93 10 121 und WO 97 19 086.

Der genannte Stand der Technik schlägt sie für therapeutische Mittel vor. In PNAS USA, 95 (1998) 1369 - 1374 werden Epothilone als nützliche therapeutische Mittel bezeichnet. Infolge ihrer therapeutischen Effekte werden nach Angew. Chem. Int. Ed., 36 (1997) 2097 - 2103 sogar eine umfangreiche Bibliothek derartiger Verbindungen (extensive library of compounds) vorgesehen.

Die Erfindung betrifft ein Verfahren zur Herstellung von Epothilon-N-oxiden, bei dem man ungeschützte Epothilone A oder B in an sich bekannter Weise in ein N-Oxid überführt.

Dieses erfindungsgemäße Verfahren kann dadurch gekennzeichnet sein, daß man die N-Oxidierung mit Persäure oder einem Dioxiran durchführt.

Ferner betrifft die Erfindung Epothilon N-Oxid (Epothilon A-N-oxid der Formel: 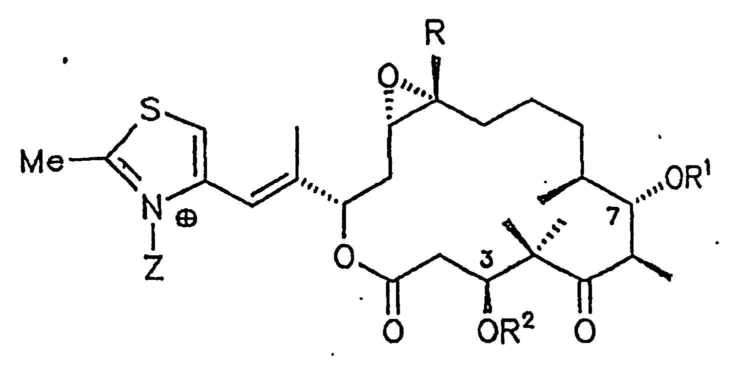 R¹, R² = H, Z = O⁻, R = H

Ferner betrifft die Erfindung Epothilon N-Oxid (Epothilon B-N-oxid), das dadurch erhältlich ist, dass man 3,7-ungeschütztes Epothilon B in ein sich bekannter Weise in ein N-Oxid überführt.

**Epothilon A-*N*-oxid (1):** Zu 100 mg Epothilon A in 1 ml Dichlormethan werden 100 mg 70%ige *m*-Chlorperbenzoesäure in 0.5 ml Dichlormethan gegeben. Nach 6-stündigem Rühren bei Raumtemperatur wird mit Dichlormethan verdünnt und nacheinander mit Natruiumsulfitlösung zur Zerstörung von überschüssiger Persäure und Natriumbicarbonatlösung ausgeschüttelt. Das Lösungsmittel wird i. Vak abgedampft, der Rückstand durch präparative HPLC an einer Nucleosil RP-18 Säule ( 250 x 20 mm, Laufmittel Methanol/Wasser 60 :40 ) aufgetrennt. Ausbeute 60 mg farbloses Öl.
R_{f}- 0.60 ( Kieselgel DC Alufolie, Laufmittel Dichlormethan/Methanol ):1);
ESI-MS (neg. Ionen) m/2 510;
UV (Methanol); lamda max. 240nm;
¹³C-NMR(CDCL₈): C-1 70,5, C-2 39,9, C-3 70,8, C-4 55.1, C-5 221.4, C-6 40.9, C-7 72.9, C-8 37.6, C-9 31.8, C-10 22.8, C-11 28.0, C-12 58.0, C-13 55.8, C-14 32.2, C-15 75.5, C-16 144,5, C-17 111.4, C-18 143.4, C-19 110.3, C-20 145.6, C-21 13.5, C-22 15.4, C-23 23.3, C-24 12.0, C-25 16.5, C-27 18.2 ppm;

### Synthesebeispiel 1

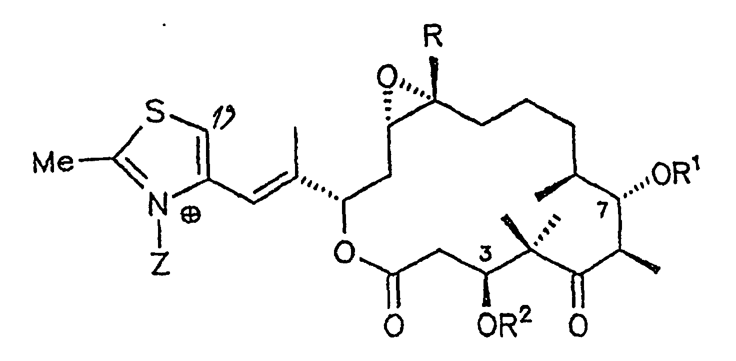

R¹, R² = H, Z = O⁻, R = H

## Patentansprüche

1. Verfahren zur Herstellung von Epothilon-N-oxiden, bei dem man 3,7-ungeschützte Epothilone A oder B in an sich bekannter Weise in ein N-Oxid überführt.

2. Verfahren nach Anspruch 1, **dadurch gekennzeichnet, daß** man die N-Oxidierung mit Persäure oder einem Dioxiran durchführt.

3. Epothilon-N-oxid (Epothilon A-N-oxid) der Formel: 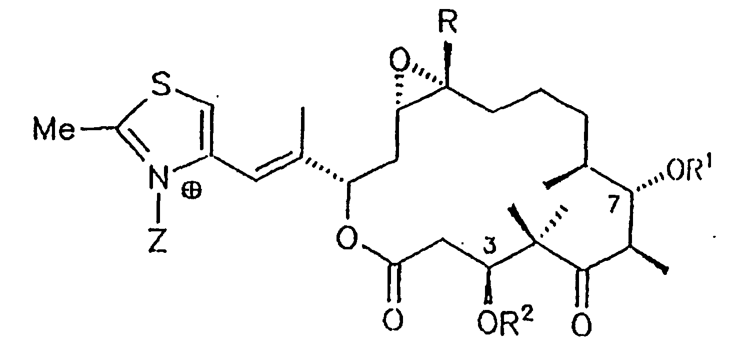 R¹, R² = H, Z = O⁻, R = H

4. Epothilon-N-oxid (Epothilon B-N-oxid), dadurch erhältlich, daß man 3,7-ungeschütztes Epothilon B in an sich bekannter Weise in ein N-Oxid überführt.

## Claims

1. Process for the preparation of epothilone N-oxides in which 3,7-unprotected epothilones A or B are converted into an N-oxide in a manner known per se.

2. Process according to claim 1, **characterized in that** N-oxidation is performed with peracid or a dioxirane.

3. Epothilone-N-oxide (epothilone A-N-oxide) of the following formula: 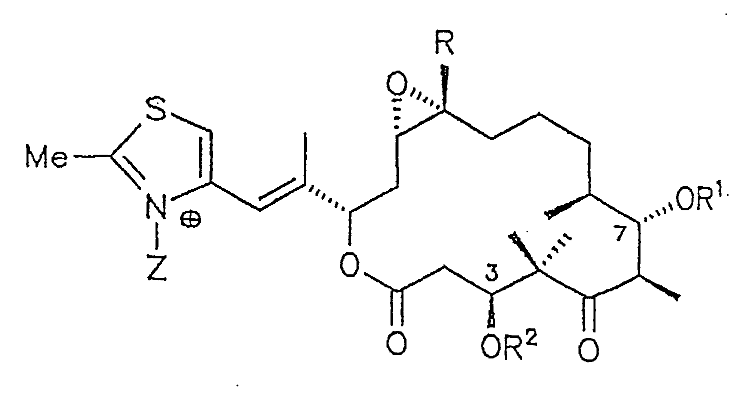 R¹, R² = H, Z = O⁻, R = H

4. Epothilone N-oxide (epothilone B-N-oxide), obtainable by transforming 3,7-unprotected epothilone B into an N-oxide in a manner known per se.

## Revendications

1. Procédé de préparation d'épothilones N-oxydées, dans lequel on convertit les épothilones A et B, non protégées en position 3 et 7, de manière connue en N-oxyde.

2. Procédé selon la revendication 1, **caractérisé par le fait que** l'oxydation de l'atome d'azote se fait avec un peracide ou un dioxirane.

3. Epothilone N-oxydée (Epothilone A N-oxydée) de formule : 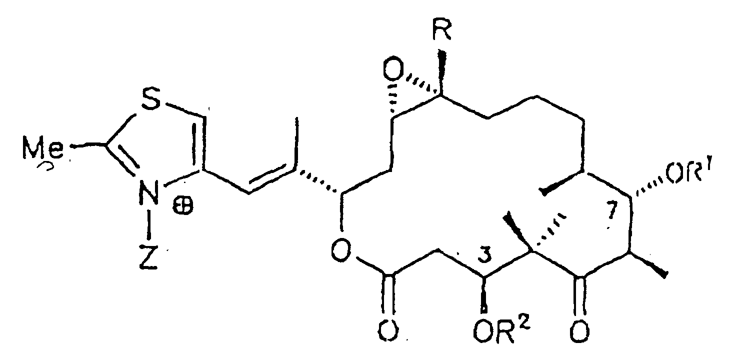 dans laquelle R¹ et R² représentent un atome d'hydrogène, Z représente un O⁻, et R un atome d'hydrogène.

4. Epothilone N-oxydée (Epothilone B N-oxydée) que l'on peut obtenir en convertissant l'épothilone B non protégée en position 3 et 7 d'une manière connue en soi en N-oxyde.
